# EUROPÄISCHE PATENTSCHRIFT

(11) **EP 2 543 317 B1**
(45) Veröffentlichungstag und Bekanntmachung des Hinweises auf die Patenterteilung: **12.10.2016**
(21) Anmeldenummer: 11172948.9
(22) Anmeldetag: 06.07.2011
(51) Int. Cl.: A61B 5/22

(54) **Vorrichtung und Verfahren zum Erfassen der Handkraft oder des Handdrucks**
Method and device for recording hand strength or hand pressure
Dispositif et procédé de détection de la force ou de la pression manuelle

(43) Veröffentlichungstag der Anmeldung: 09.01.2013
(73) Patentinhaber: MSYS AG, 6052 Hergiswil (CH)
(72) Erfinder: Schuurmans Stekhoven, Marco, 8008 Zürich (CH); Brinkhaus, Bernhard, 8955 Oetwil an der Limmat (CH)
(74) Vertreter: Dr. Graf & Partner AG

(56) Entgegenhaltungen:
- WO-A1-2011/080237
- WO-A2-2009/052100
- US-A1- 2004 243 021
- US-A1- 2006 063 647

## Beschreibung

Die Erfindung betrifft eine Vorrichtung zum Erfassen der Handkraft oder des Handdrucks und insbesondere der Schmerzempfindung gemäss dem Oberbegriff von Anspruch 1. Die Erfindung betrifft weiter ein Verfahren zum Erfassen der Handkraft oder des Handdrucks gemäss Anspruch 13.

### Stand der Technik

Schmerzen gehören zu den häufigsten Gründen einen Arzt aufzusuchen, wobei der Schmerz eine subjektive Sinnesempfindung darstellt, welche sensorische, kognitive und emotionale Aspekte beinhaltet. Eine genaue Schmerzmessung wäre deshalb äusserst hilfreich, zum Beispiel für eine Schmerzüberwachung, eine Diagnose, oder zum Anpassen einer medikamentösen Schmerztherapie.

Die Druckschrift WO 2009/052100 offenbart eine Vorrichtung zum Messen von Schmerzen. Diese Vorrichtung weist den Nachteil auf, dass der Schmerz nur äusserst ungenau messbar ist, und dass die Vorrichtung nur geeignet ist um Schmerzen im Darm zu messen. Die Druckschrift US2006/0063647A1 offenbart eine Vorrichtung zur Messung der Muskelkraft, welche die Merkmale des Oberbegriffs von Anspruch 1 umfasst.

### Darstellung der Erfindung

Es hat sich gezeigt, dass eine genaue Erfassung der Handkraft beziehungsweise des Handrucks interessante Möglichkeiten zur Überwachung und Behandlung von Menschen ermöglicht, und dass die genaue Erfassung der Handkraft beziehungsweise des Handdrucks insbesondere zum Erfassen von Schmerzempfindungen eines Menschen geeignet ist. Eine Vorrichtung sowie Verfahren zum Erfassen und Messen von Schmerzen eines Menschen sind im Dokument WO2011/080237 (Anmeldung PCT/EP2010/070736) mit dem Titel "Vorrichtung und Verfahren zum Erfassen und Messen von Schmerzen" offenbart. Dies Dokument fällt unter Art 54(3) EPÜ.

Aufgabe der vorliegenden Erfindung ist es eine vorteilhaftere Vorrichtung zum Erfassen der Handkraft oder des Handdrucks und insbesondre von Schmerzempfindungen zu bilden.

Diese Aufgabe wird gelöst mit einer Vorrichtung umfassend die Merkmale von Anspruch 1. Die Unteransprüche 2 bis 11 beziehen sich auf weitere, vorteilhafte Ausgestaltungen. Diese Aufgabe wird weiter gelöst mit einem Verfahren umfassend die Merkmale von Anspruch 13.

Die Aufgabe wird insbesondere gelöst mit einer Vorrichtung zum Erfassen der Handkraft oder des Handdrucks und insbesondere von Schmerzempfindungen eines Menschen, umfassend eine Druck- oder Kraftmessvorrichtung, sowie umfassend einen sich in einer Längsrichtung erstreckenden Hohlkörper, wobei der Hohlkörper ein oberes festes Endteil, ein unteres festes Endteil sowie ein Distanzhalteelement umfasst, wobei das obere Endteil und das untere Endteil über das Distanzhalteelement gegenseitig beabstandet gehalten sind, wobei sich das Distanzhalteelement in Längsrichtung erstreckt, und wobei der Hohlkörper eine flexible Aussenhülle umfasst, welche das obere Endteil derart mit dem unteren Endteil verbindet, dass sich ein geschlossener Innenraum ausbildet innerhalb welchem auch das Distanzhalteelement angeordnet ist, wobei die Aussenhülle derart ausgestaltet ist, dass diese von einer Hand zumindest teilweise umschliessbar ist, und wobei der Innenraum des Hohlkörpers ein Gel-, ein elastisches Mehrkomponenten- oder ein Flüssigkeitsmaterial enthält, welches als Druckmittler wirkt, und wobei die Druck- oder Kraftmessvorrichtung in Längsrichtung zumindest teilweise im Innenraum verläuft, um den Druck von der Aussenhülle über den Druckmittler auf die Druck- oder Kraftmessvorrichtung zu übertragen, und umfassend einen Schmerzaktuator, ausgestaltet als eine Wärmeerzeugungsvorrichtung, wobei der Schmerzaktuator eine metallische Abdeckung umfasst, an deren Unterseite ein Heizelement sowie ein Temperatursensor angeordnet ist.

Die Aufgabe wird weiter insbesondere gelöst mit einem Verfahren zum Erfassen von Schmerzempfindungen oder Schmerzsensitivität eines Menschen, indem mit einer Hand ein zylinderförmiger Hohlkörper zumindest teilweise umschlossen wird, indem durch Zudrücken der Hand ein Druck auf eine flexible Aussenhülle des Hohlkörpers ausgeübt wird, wobei der Druck über ein Gel-, ein elastisches Mehrkomponenten- oder ein Flüssigkeitsmaterial, welches als Druckmittler wirkt, auf eine im Hohlkörper angeordnete Druck- oder Kraftmessvorrichtung übertragen wird, wobei das elastische Verhalten der flexiblen Aussenhülle über den Druck des Druckmittlers derart eingestellt wird, dass der Durchmesser der Aussenhülle während dem Drücken beibehalten oder im Wesentlichen beibehalten wird, und dass der von der Druck- oder Kraftmessvorrichtung gemessene Druck oder die gemessene Kraft als Mass für den vom Menschen empfundene Schmerz verwendet wird, und dass ein sich zunehmend erwärmender Schmerzaktuator berührt wird, welcher eine metallische Abdeckung umfasst, an deren Unterseite ein Heizelement sowie ein Temperatursensor angeordnet ist, und dass der gemessene Druck oder die gemessene Kraft in Funktion der Temperatur des Schmerzaktuators erfasst wird.

Die erfindungsgemässe Vorrichtung bezieht sich auf das Gebiet der Erfassung und Messung der Handkraft oder des Handdrucks, und insbesondere auf das Gebiet der Messung von Schmerzen und Schmerzempfindungen eines Menschen und deren Erfassung. Die erfindungsgemässe Vorrichtung ermöglicht es eine Handkraft oder einen Handdruck genau und reproduzierbar zu messen, wobei die gemessenen Werte besonders vorteilhafterweise als ein Mass für Schmerz beziehungsweise für Schmerzempfinden geeignet sind.

Die erfindungsgemässe Vorrichtung ermöglicht es zudem auch Schmerzen zu generieren. Ein wesentlicher Vorteil der erfindungsgemässen Vorrichtung ist darin zu sehen, dass diese es ermöglicht Schmerzen reproduzierbar zu messen und deren Höhe vorzugsweise in gewisse Niveaus zu ordnen. Ein Mensch weist die Eigenschaft auf, dass dieser bei Schmerzen, insbesondere bei starken Schmerzen, die Hand reflexartig zusammenzieht, wobei sich die Handinnenfläche bis zur Ausbildung einer Faust zusammenziehen kann. Um dieses reflexartige Verhalten von Menschen zu nutzen ist die erfindungsgemässe Vorrichtung derart ausgestaltet, dass diese einen Hohlkörper aufweist, der von einer Hand zumindest teilweise umschliessbar ist. In einer besonders vorteilhaften Ausgestaltung ist der Hohlkörper rohrförmig ausgestaltet, insbesondere als geradlinig verlaufendes Rohr. Ein derartig ausgestalteter Hohlkörper liegt gut in der Hand und nutzt die natürliche, reflexartige Bewegung des Menschen zur Messung der Handkraft oder des Handdrucks beziehungsweise zur Messung von Schmerzen, in dem die Hand den Hohlkörper mit zunehmendem Schmerz auf natürliche weise stärker und kraftvoller hält. Die Fläche des Hohlkörpers, welche zur Anlage an der Hand bestimmt ist, sollte vorzugsweise formstabil oder im Wesentlichen formstabil ausgestaltet sein, aber sich auch angenehm anfühlen, was den Vorteil ergibt, dass sich dessen Form unabhängig von der angreifenden Kraft der Hand nicht oder nur sehr geringfügig ändert. Dies ermöglicht die von der Hand bewirkten Kraft reproduzierbar und genau zu messen. Anders ausgedrückt, würde sich die Form des Hohlkörpers auf Grund der von der Hand auf den Hohlkörper bewirkten Kraft ausgeprägt verändern, was zum Beispiel bei einem mit Luft gefüllten Gummibalg der Fall wäre, so wäre der empfundene Schmerz nicht mehr über die von der Hand bewirkten Kraft messbar, da der Gummibalg bei zunehmender Kraft nachgibt und dessen Form verändern, sodass es schwierig oder nicht mehr möglich ist den empfundenen Schmerz über die Kraft vorzugsweise linear auszudrücken, insbesondere wenn die Hand bereits zur Faust geballt ist. Es kann sich als vorteilhaft erweisen die Oberfläche des Hohlkörpers derart zu gestalten, dass sich an der Handfläche, welche an der erfindungsgemässen Vorrichtung anliegt, keine ausgeprägten und eventuell sogar schmerzhaften Druckstellen ausbilden. Solche Druckstellen sind unangenehm und könnten sogar die Messung verfälschen. In einer vorteilhaften Ausgestaltung weist die Oberfläche des Hohlkörpers, welche zur Anlagen an der Hand bestimmt ist, eine gewisse Elastizität auf, um an der anliegenden Hand derartige Druck- oder Schmerzpunkte zu vermeiden. Die erfindungsgemässe Vorrichtung ermöglicht es die von der Hand bewirkte Druck- oder Presskraft besonders genau zu messen. Die erfindungsgemässe Vorrichtung erlaubt es die Stärke des empfundenen Schmerz bei einer individuellen Person zuverlässig und auch reproduzierbar zu messen.

Die erfindungsgemässe Vorrichtung weist insbesondere den Vorteil auf, dass die Schmerzen und vorzugsweise auch deren Zustände genau erfassbar sind, und dass die Messungen der Schmerzen objektiv reproduzierbar sind.

Die Erfindung wird nachfolgend an Hand von Ausführungsbeispielen beschrieben.

### Kurze Beschreibung der Zeichnungen

Die zur Erläuterung der Ausführungsbeispiele verwendeten Zeichnungen zeigen:
- Fig. 1: eine Seitenansicht einer Vorrichtung zum Erfassen der Handkraft;
- Fig. 2: einen Längsschnitt durch Figur 1 entlang der Schnittlinie A-A;
- Fig. 3: eine Seitenansicht eines Distanzhalteelementes;
- Fig. 4: einen Querschnitt durch Figur 3 entlang der Schnittlinie B-B;
- Fig. 5: eine Seitenansicht einer Druckmessvorrichtung;
- Fig. 6: einen Längsschnitt durch die in Figur 5 dargestellte Druckmessvorrichtung entlang der Schnittlinie C-C;
- Fig. 7a: schematisch einen Längsschnitt durch ein weiteres Ausführungsbeispiel eines Gehäuses mit Distanzhalteelementen;
- Fig. 7b: schematisch eine Draufsicht auf Figur 7a;
- Fig. 8: schematisch eine Seitenansicht einer weiteren Druckmessvorrichtung;
- Fig. 9: schematisch ein weiteres Ausführungsbeispiel einer Vorrichtung zum Erfassen der Handkraft mit einer diese umschliessenden Hand;
- Fig. 10: einen Längsschnitt durch einen Schmerzaktuator.

Grundsätzlich sind in den Zeichnungen gleiche Teile mit gleichen Bezugszeichen versehen.

### Wege zur Ausführung der Erfindung

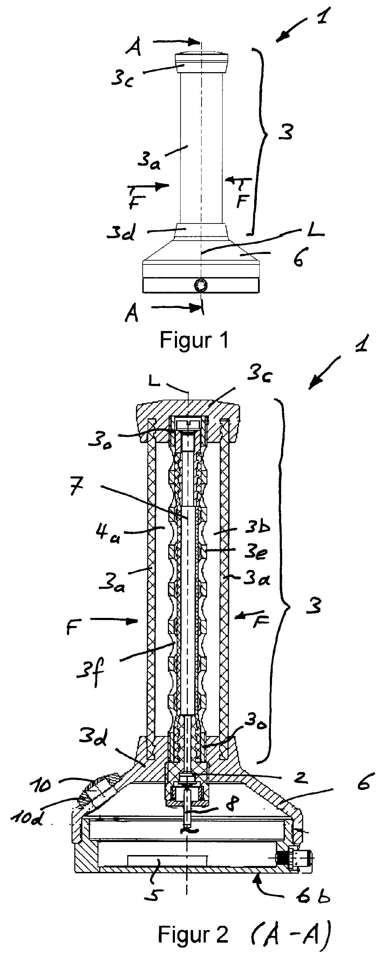

Fig. 1 zeigt in einer Seitenansicht und Figur 2 in einem Längsschnitt entlang der Schnittlinie A-A eine Vorrichtung 1 zum Erfassen der Handkraft oder des Handdrucks und insbesondere der Schmerzempfindung. Die Vorrichtung 1 umfasst einen sich in einer Längsrichtung L erstreckenden Hohlkörper 3, wobei der Hohlkörper 3 ein oberes festes Endteil 3c, ein unteres festes Endteil 3d sowie ein Distanzhalteelement 3e umfasst, wobei das obere Endteil 3c und das untere Endteil 3d über das Distanzhalteelement 3e gegenseitig beabstandet gehalten sind, wobei sich das Distanzhalteelement 3e in Längsrichtung L erstreckt. Der Hohlkörper 3 umfasst eine flexible Aussenhülle 3a, welche das obere Endteil 3c derart mit dem unteren Endteil 3d verbindet, dass sich ein geschlossener Innenraum 3b ausbildet, innerhalb welchem auch das Distanzhalteelement 3e angeordnet ist. Die Aussenhülle 3a ist derart ausgestaltet, dass diese von einer Hand zumindest teilweise umschliessbar ist, indem der Querschnitt beziehungsweise der Umfang der Aussenhülle 3a entsprechend der Grösse einer menschlichen Hand gewählt ist. Das obere und untere Endteil 3c, 3d sind von besonderer Wichtigkeit zur genauen Messung, da das obere und das untere Endteil 3c,3d fest beziehungsweise starr sind und ein Ausweichen beziehungsweise ein Vergrössern des Innenraumes 3b in Verlaufsrichtung L verhindern. Die Vorrichtung 1 umfasst zudem eine Druck- oder Kraftmessvorrichtung 7, welche in Längsrichtung L im Innenraum 3b verläuft. Der Innenraum 3b des Hohlkörpers 3 enthält beziehungsweise ist aufgefüllt mit einem Gelmaterial 4a, einem elastischen Mehrkomponentenmaterial 4a oder ein Flüssigkeitsmaterial 4a, welches als Druckmittler wirkt, um den Druck von der Aussenhülle 3a über den Druckmittler auf die Druck- oder Kraftmessvorrichtung 7 zu übertragen.

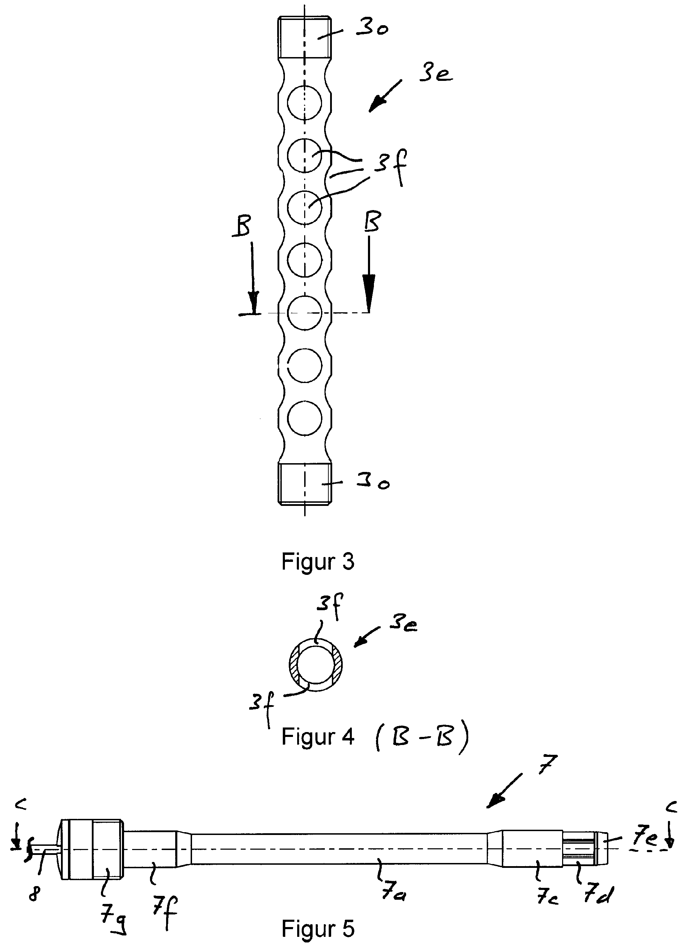

Das Distanzhalteelement 3e, welches in Figur 3 in einer Seitenansicht und in Figur 4 in einem Schnitt entlang der Schnittlinie B-B im Detail dargestellt ist, ist als Hohlrohr mit kreisförmigen Wandöffnungen 3f ausgestaltet, und umfasst oben und unten einen Befestigungsabschnitt 3o, welche, wie in Figur 2 dargestellt, mit dem oberen Endteil 3c beziehungsweise dem unteren Endteil 3d fest verbunden sind, um die beiden Endteile 3c,3d in einer gegenseitigen definierten Distanz zu halten.

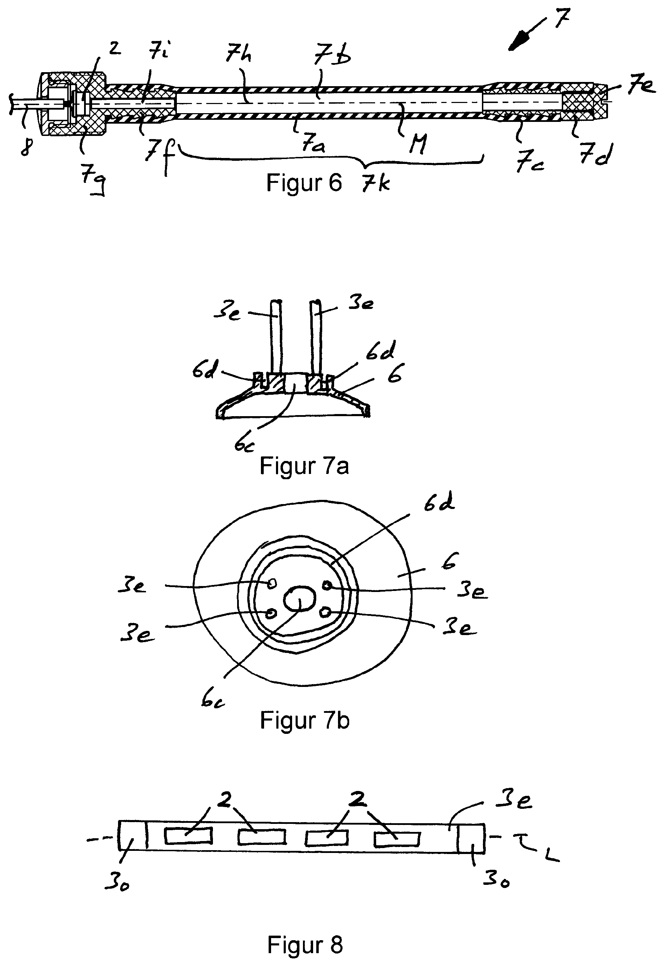

Wie in Figur 2 dargestellt ist die Druck- oder Kraftmessvorrichtung 7 von unten durch das untere Endteil 3d in den Innenraum des Distanzhalteelementes 3e eingeführt, wobei die Druck- oder Kraftmessvorrichtung 7 mit dem unteren Endteil 3d verschraubt ist und dadurch fest gehalten ist. Die Druck- oder Kraftmessvorrichtung 7 ist in den Figuren 5 und 6 im Detail dargestellt, wobei Figur 5 eine Seitenansicht zeigt und Figur 6 einen Schnitt entlang der Schnittlinie C-C. Wie aus Figur 6 ersichtlich umfasst die Druck- und Kraftmessvorrichtung 7 einen in Verlaufsrichtung M verlaufenden, flexiblen Hohlkörper 7a mit Innenraum 7b, wobei der flexible Hohlkörper 7a rechts einen oberen Endabschnitt 7c aufweist, der mit einem oberen Abschluss 7d fest und vorzugsweise Fluid dicht verbunden ist. Die Öffnung des oberen Abschlusses 7d ist mit einer Schraube 7e verschlossen. Der flexible Hohlkörper 7a weist links einen unteren Endabschnitt 7f auf, der mit einem unteren Abschluss 7g fest und vorzugsweise Fluid dicht verbunden ist. Ein Kraftaufnehmer 2 ist im unteren Abschluss 7g angeordnet, wobei der untere Abschluss 7g einen Fluid leitenden Kanal 7i aufweist, welcher den Innenraum 7b mit dem Kraftaufnehmer 2 verbindet. Der Innenraum 2b sowie der Fluid leitende Kanal 2i ist mit einem zweiten Flüssigkeitsmaterial 7h gefüllt. Der Kraftaufnehmer 2 weist eine senkrecht zur Verlaufsrichtung M verlaufende Oberfläche auf, an welcher das zweite Flüssigkeitsmaterial 7h anliegt, sodass der Kraftaufnehmer 2 senkrecht zur Verlaufsrichtung M zum Innenraum 7b angekoppelt ist, um den Druck des zweiten Flüssigkeitsmaterials 7h zu messen. Der Kraftaufnehmer 2 ist über ein Kabel 8 mit der in Figur 2 dargestellten Elektronikeinheit 5 verbunden. Die Wand des flexiblen, schlauchförmigen Hohlkörpers 7a überträgt ein aussen entlang des Abschnittes 7k anliegende Druckkraft auf die sich im Innenraum 7b befindliche Flüssigkeit 7h, wobei der Kraftaufnehmern 2 den Druck beziehungsweise die durch die Flüssigkeit 7h auf den Kraftaufnehmer 2 bewirkte Kraft misst. Der Hohlkörper 7a kann die Kraft nur entlang des Abschnittes 7k von aussen nach innen übertragen, da der Hohlkörper 7a entlang des oberen Endabschnittes 7c und entlang des unteren Endabschnittes 7f am oberen Abschluss 7d beziehungsweise am unteren Abschluss 7g anliegt. Die Schraube 7e dient unter anderem dazu den Innenraum 7b vollständig mit der Flüssigkeit 7h zu füllen und den Innenraum 7b danach wieder Fluid dicht zu verschliessen. In einer vorteilhaften Ausgestaltung weist der Abschnitt 7k des flexiblen Hohlkörpers 7a, insbesondere bedingt durch das zweite Flüssigkeitsmaterial 7h, eine Shore-Härte im Bereich zwischen 10 und 20 auf. Als Flüssigkeitsmaterial 7h wird beispielsweise ein Öl verwendet.

In einer bevorzugten Ausgestaltung verläuft die Kraftmessvorrichtung 7, wie in Figur 2 dargestellt, entlang der gesamten Länge L des Innenraumes 3b und zudem noch entlang des untern Endteils 3d, wobei sich der Abschnitt 7k nur innerhalb des Innenraumes 3b verläuft. In einer weiteren Ausgestaltung könnte die Kraftmessvorrichtung 7 auch derart ausgestaltet sein, dass diese nicht entlang der gesamten Länge L des Innenraume 3b verläuft, sondern zum Beispiel nur zur Hälfte der Länge L, oder beispielsweise zu Dreiviertel der Länge L. In der am meisten bevorzugten Ausgestaltung verläuft die Kraftmessvorrichtung 7, wie in Figur 2 dargestellt, entlang der Mitte beziehungsweise entlang der Achse L. Die Kraftmessvorrichtung 7 ist wie dargestellt vorzugsweise bezüglich der Längsachse zentriert angeordnet, damit die an der flexiblen Aussenhülle 3a anliegenden Kräfte gleichmässig auf die Druck- oder Kraftmessvorrichtung 7 übertragen werden. Die Druck- oder Kraftmessvorrichtung 7 könnte jedoch auch exzentrisch verlaufend im Innenraum 7b angeordnet sein.

In einer besonders vorteilhaften Ausgestaltung ist die flexible Aussenhülle 3a, wie in Figur 1 und 2 dargestellt, hohlzylinderförmig ausgestaltet. Die flexible Aussenhülle 3a ist vorzugsweise aus Silikon, Gummi oder Kautschuk gefertigt. Das als Druckmittler wirkende Gelmaterial 4a, das elastische Mehrkomponentenmaterial 4a oder das Flüssigkeitsmaterial 4a überträgt den Druck von der Aussenhülle 3a auf die Druck- oder Kraftmessvorrichtung 7. Der Druckmittlers wird beim Einfüllen in den Innenraum 3b vorteilhafterweise mit einem vorherbestimmten Druck eingefüllt, sodass der Druckmittler in einem Ruhezustand, das heisst ohne eine an der Aussenhülle 3a angreifenden Kraft, einen vorherbestimmten Druck aufweist. Der vorherbestimmte Fülldruck des Druckmittlers beeinflusst die Härte beziehungsweise die Nachgiebigkeit der flexiblen Aussenhülle 3a. In einer besonders vorteilhaften Ausgestaltung ist die flexible Aussenhülle 3a aus einem derartigen Material gewählt und/oder ist der vorherbestimmte Druck des Druckmittlers derart gewählt, dass die flexible Aussenhülle 3a eine Shore-Härte im Bereich zwischen 20 und 90 aufweist. Damit lässt sich unter anderem folgendes erzielen: Einerseits sollte sich die flexible Aussenhülle 3a für die anliegende Hand angenehm anfühlen, was dadurch erzielt wird, dass die flexible Aussenhülle 3a beziehungsweise der Druckmittler gewisse elastische Eigenschaften aufweist. Diese elastischen, von der Hand als angenehm empfundenen Eigenschaften weisen den Vorteil auf, dass sich an der Hand keine Druckstellen ergeben. Eine harte Aussenhülle 3a würde an einer sich zusammenpressenden Hand Druckstellen bewirken, was zur Folge hätte, dass die Hand auf Grund des auftretenden Schmerzes oder Druckes teilweise wieder entspannt wird beziehungsweise dass die Kraft reduziert wird. Das Vermeiden solcher Druckstellen ist daher für eine genaue Messung der Handkraft, des Handdrucks oder von Schmerzempfindungen von entscheidender Bedeutung. Andererseits ist es besonders vorteilhaft, wenn sich der Durchmesser der flexiblen Aussenhülle 3a selbst bei grösseren angreifenden Kräften nur geringfügig ändert, weil es für die an der Aussenhülle 3a anliegende Hand schwieriger wird eine grosse Kraft auf die Aussenhülle 3a zu bewirken je kleiner der Durchmesser der flexiblen Aussenhülle 3a wird. Die erfindungsgemässe Vorrichtung weist in einer besonders vorteilhaften Ausgestaltung somit den Vorteil auf, dass über den Fülldruck des Druckmittlers die vorhin genannten Eigenschaften der Aussenhülle 3a einstellbar beziehungsweise vorher bestimmbar sind.

Figur 2 zeigt zudem ein Gehäuse 6 mit einer Standfläche 6b, wobei der untere feste Abschnitt 3d Teil des Gehäuses 6 bildet. Der untere feste Abschnitt 3d ist derart angeordnet, dass der Hohlkörper 3 im Wesentlichen senkrecht zur Standfläche 6b verläuft. Im Gehäuse 6 ist vorzugsweise zudem eine Elektronikeinheit 5 angeordnet, welche über das Kabel 8 mit dem Kraftaufnehmer 2 verbunden ist. Im Gehäuse 6 ist vorzugsweise auch ein Schmerzaktuator 10 angeordnet, welcher als eine Wärmeerzeugungsvorrichtung ausgestaltet ist.

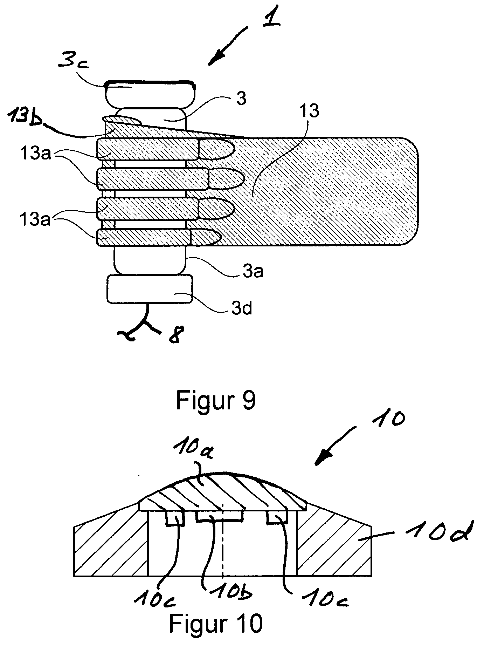

Figur 10 zeigt in einem Längsschnitt einen Schmerzaktuator 10 mit einer metallischen Abdeckung 10a, an deren Unterseite ein Heizelement 10c sowie ein Temperatursensor 10b angeordnet sind. Die elektrischen Leitungen zur Elektronikeinheit 5 sind nicht dargestellt.

Figur 7a zeigt schematisch und teilweise nur angedeutet einen Schnitt durch und Figur 7b eine Draufsicht auf ein Gehäuse 6 mit Bohrung 6c und Ausnehmung 6d. Das Distanzhalteelement 3e ist im dargestellten Beispiel aus vier Stangen gebildet, welchen in Verlaufsrichtung L verlaufen und das oberen Endteil 3c mit dem unteren Endteil 3d verbinden. Das Distanzhalteelement 3e kann in einer Vielzahl von Möglichkeiten gefertigt sein, um diese Distanzhaltung zu bewirken.

Figur 8 zeigt schematisch ein Distanzhalteelement 3e sowie eine Druck- oder Kraftmessvorrichtung 7, indem eine Mehrzahl von Kraftaufnehmern 2 in Längsrichtung L gegenseitig beabstandet am Distanzhalteelement 3e angeordnet sind. Jeder Kraftaufnehmer 2 ist Signal leitend mit der Elektronikeinheit verbunden, sodass der vom Druckmittler im Innenraum 3b anliegende Druck messbar ist.

Figur 9 zeigt schematisch ein weiteres Ausführungsbeispiel der erfindungsgemässen Vorrichtung 1, umfassend einen Hohlkörper 3 mit oberem und unterem Endteil 3c,3d und dazwischen angeordneter flexibler Aussenhülle 3a, wobei die Vorrichtung 1, im Unterschied zu der in Figur 2 dargestellten Vorrichtung 1, kein Gehäuse 6 aufweist, und das Kabel 8 einer nachgeordneten Signalauswertevorrichtung zugeleitet ist. Figur 9 zeigt zudem schematisch eine Hand 13 mit Fingern 13a und Daumen 13b, welche die flexible Aussenhülle 3a umschliessen.

Das Verfahren zum Erfassen von Schmerzempfindungen oder Schmerzsensitivität eines Menschen erfolgt vorteilhafterweise, indem mit einer Hand ein zylinderförmiger Hohlkörper 3 zumindest teilweise umschlossen wird, indem durch Zudrücken der Hand ein Druck auf eine flexible Aussenhülle 3a des Hohlkörpers 3 ausgeübt wird, wobei der Druck über ein Gel-, ein elastisches Mehrkomponenten- oder ein Flüssigkeitsmaterial 4a,4b, welches als Druckmittler wirkt, auf eine im Hohlkörper 3 angeordnete Druck- oder Kraftmessvorrichtung 7 übertragen wird, wobei das elastische Verhalten der flexiblen Aussenhülle 3a über den Druck des Druckmittlers derart eingestellt wird, dass der Durchmesser der Aussenhülle 3a während dem Drücken beibehalten oder im Wesentlichen beibehalten wird, und dass der von der Druck- oder Kraftmessvorrichtung 7 gemessene Druck oder die gemessene Kraft als Mass für den vom Menschen empfundene Schmerz verwendet wird, und dass ein sich zunehmend erwärmender Schmerzaktuator 10 berührt wird, und dass der gemessene Druck oder die gemessene Kraft in Funktion der Temperatur des Schmerzaktuators 10 erfasst wird. Die Temperaturerhöhung erfolgt vorteilhafterweise insgesamt um einen vorherbestimmten Betrag im Bereich von 1 bis 5 °C.

## Patentansprüche

1. Vorrichtung (1) zum Erfassen der Handkraft oder des Handdrucks und insbesondere von Schmerzempfindungen eines Menschen, umfassend eine Druck- oder Kraftmessvorrichtung (7), sowie umfassend einen sich in einer Längsrichtung (L) erstreckenden Hohlkörper (3), wobei der Hohlkörper (3) ein oberes festes Endteil (3c), ein unteres festes Endteil (3d) sowie ein Distanzhalteelement (3e) umfasst, wobei das obere Endteil (3c) und das untere Endteil (3d) über das Distanzhalteelement (3e) gegenseitig beabstandet gehalten sind, wobei sich das Distanzhalteelement (3e) in Längsrichtung (L) erstreckt, und wobei der Hohlkörper (3) eine flexible Aussenhülle (3a) umfasst, welche das obere Endteil (3c) derart mit dem unteren Endteil (3d) verbindet, dass sich ein geschlossener Innenraum (3b) ausbildet innerhalb welchem auch das Distanzhalteelement (3e) angeordnet ist, wobei die Aussenhülle (3a) derart ausgestaltet ist, dass diese von einer Hand zumindest teilweise umschliessbar ist, und wobei der Innenraum (3b) des Hohlkörpers (3) ein Gel-, ein elastisches Mehrkomponenten- oder ein Flüssigkeitsmaterial (4a) enthält, welches als Druckmittler wirkt, und wobei die Druck- oder Kraftmessvorrichtung (7) in Längsrichtung (L) zumindest teilweise im Innenraum (3b) verläuft, um den Druck von der Aussenhülle (3a) über den Druckmittler auf die Druck- oder Kraftmessvorrichtung (7) zu übertragen, **dadurch gekennzeichnet, dass** die Vorrichtung (1) einen Schmerzaktuator (10) umfasst, der als eine Wärmeerzeugungsvorrichtung ausgestaltet ist, wobei der Schmerzaktuator (10) eine metallische Abdeckung (10a) umfasst, an deren Unterseite ein Heizelement (10c) sowie ein Temperatursensor (10b) angeordnet ist.

2. Vorrichtung (1) nach Anspruch 1, **dadurch gekennzeichnet,**
**dass** die Kraftmessvorrichtung (7) zumindest entlang der gesamten Länge (L) des Innenraumes (3b) verläuft.

3. Vorrichtung nach einem der vorhergehenden Ansprüche,
**dadurch gekennzeichnet, dass** die Druck- oder Kraftmessvorrichtung (7) entlang der Mitte des Hohlkörpers (3) verläuft.

4. Vorrichtung nach einem der vorhergehenden Ansprüche,
**dadurch gekennzeichnet, dass** das Distanzhalteelement (3e) als Hohlrohr mit Wandöffnungen (3f) ausgestaltet ist, dass das Distanzhalteelement (3e) entlang der Mittelachse des Hohlkörpers (3) verläuft, und dass die Druck- oder Kraftmessvorrichtung (7) innerhalb des Distanzhalteelementes (3e) verlaufend angeordnet ist.

5. Vorrichtung nach einem der vorhergehenden Ansprüche,
**dadurch gekennzeichnet, dass** die flexible Aussenhülle (3a) hohlzylinderförmig ausgestaltet ist.

6. Vorrichtung nach einem der vorhergehenden Ansprüche,
**dadurch gekennzeichnet, dass** über den Druck des Druckmittlers die Härte beziehungsweise die Nachgiebigkeit der flexiblen Aussenhülle (3a) bestimmbar ist.

7. Vorrichtung nach Anspruch 6, **dadurch gekennzeichnet, dass** die flexible Aussenhülle (3a) eine Shore-Härte im Bereich zwischen 20 und 90 aufweist.

8. Vorrichtung nach einem der vorhergehenden Ansprüche,
**dadurch gekennzeichnet, dass** die Druck- oder Kraftmessvorrichtung (7) einen Kraftaufnehmer (2) sowie einen geradlinig in Verlaufsrichtung (M) verlaufenden, flexiblen Hohlkörper (7a) mit einem Innenraum (7b) umfasst, dass der Innenraum (7b) des Hohlkörpers der Druck- oder Kraftmessvorrichtung geschlossen ist und ein zweites Flüssigkeitsmaterial (7h) enthält, und dass der Kraftaufnehmer (2) senkrecht zur Verlaufsrichtung (M) zum Innenraum (7b) des Hohlkörpers der Druck- oder Kraftmessvorrichtung angekoppelt ist, um den Druck des zweiten Flüssigkeitsmaterials (7h) zu messen.

9. Vorrichtung nach einem der Ansprüche 1 bis 7, **dadurch gekennzeichnet, dass** die Druck- oder Kraftmessvorrichtung (7) eine Mehrzahl von Kraftaufnehmern (2) umfasst, welche in Längsrichtung (L) gegenseitig beabstandet am Distanzhalteelement (3e) angeordnet sind.

10. Vorrichtung nach einem der vorhergehenden Ansprüche,
**dadurch gekennzeichnet, dass** der untere feste Abschnitt (3d) Teil eines Gehäuses (6) bildet, dass das Gehäuse (6) eine Standfläche (6b) aufweist, und dass der untere feste Abschnitt (3d) derart angeordnet ist, dass der Hohlkörper (3) im Wesentlichen senkrecht zur Standfläche (6b) verläuft.

11. Vorrichtung nach Anspruch 1, **dadurch gekennzeichnet, dass** der Schmerzaktuator (10) am Gehäuse (6) angeordnet ist.

12. Verwendung einer Vorrichtung nach einem der vorhergehenden Ansprüche zum Erfassen von Schmerzempfindungen oder Schmerzsensitivität eines Menschen.

13. Verfahren zum Erfassen von Schmerzempfindungen oder Schmerzsensitivität eines Menschen, indem mit einer Hand ein zylinderförmiger Hohlkörper (3) zumindest teilweise umschlossen wird, indem durch Zudrücken der Hand ein Druck auf eine flexible Aussenhülle (3a) des Hohlkörpers (3) ausgeübt wird, wobei der Druck über ein Gel-, ein elastisches Mehrkomponenten- oder ein Flüssigkeitsmaterial (4a,4b), welches als Druckmittler wirkt, auf eine im Hohlkörper (3) angeordnete Druck- oder Kraftmessvorrichtung (7) übertragen wird, wobei das elastische Verhalten der flexiblen Aussenhülle (3a) über den Druck des Druckmittlers derart eingestellt wird, dass der Durchmesser der Aussenhülle (3a) während dem Drücken beibehalten oder im Wesentlichen beibehalten wird, und dass der von der Druck- oder Kraftmessvorrichtung (7) gemessene Druck oder die gemessene Kraft als Mass für den vom Menschen empfundenen Schmerz verwendet wird, und dass ein sich zunehmend erwärmender Schmerzaktuator (10) berührt wird, welcher eine metallische Abdeckung umfasst, an deren Unterseite ein Heizelement sowie ein Temperatursensor angeordnet ist, und dass der gemessene Druck oder die gemessene Kraft in Funktion der Temperatur des Schmerzaktuators (10) erfasst wird.

## Claims

1. An apparatus (1) for detecting the hand force or the hand pressure and in particular perceptions of pain of a person, comprising a pressure or force measuring apparatus (7) and comprising a hollow body (3) extending in a longitudinal direction (L), wherein the hollow body (3) includes an upper fixed end part (3c), a lower fixed end part (3d) and a spacer element (3e), wherein the upper end part (3c) and the lower end part (3d) are kept mutually spaced apart via the spacer element (3e), wherein the spacer element (3e) extends in the longitudinal direction (L), and wherein the hollow body (3) includes a flexible outer cover (3a) which connects the upper end part (3c) to the lower end part (3d) such that a closed inner space (3b) is formed within which the spacer element (3e) is also arranged, wherein the outer cover (3a) is designed such that it can be at least partly surrounded by a hand, and wherein the inner space (3b) of the hollow body (3) includes a gel material, an elastic multicomponent material or a liquid material (4a) which acts as a pressure transmitter, and wherein the pressure or force measuring apparatus (7) extends at least partly in the inner space (3b) in the longitudinal direction (L) to transmit the pressure from the outer cover (3a) via the pressure transmitter to the pressure or force measuring apparatus (7), **characterized in that** the apparatus (1) comprises a pain actuator (10), designed as a heat generation apparatus, wherein the pain actuator (10) includes a metallic cover (10a) at whose lower side a heating element (10c) and a temperature sensor (10b) are arranged.

2. An apparatus (1) in accordance with claim 1, **characterized in that** the force measuring apparatus (7) extends at least along the total length (L) of the inner space (3b).

3. An apparatus in accordance with one of the preceding claims, **characterized in that** the pressure or force measuring apparatus (7) extends along the center of the hollow body (3).

4. An apparatus in accordance with any one of the preceding claims, **characterized in that** the spacer element (3e) is designed as a hollow tube with wall openings (3f), **in that** the spacer element (3e) extends along the center axis of the hollow body (3), and **in that** the pressure or force measuring apparatus (7) is arranged extending within the spacer element (3e).

5. An apparatus in accordance with any one of the preceding claims, **characterized in that** the flexible outer cover (3a) is designed in the form of a hollow cylinder.

6. An apparatus in accordance with any one of the preceding claims, **characterized in that** the hardness or the resilience of the flexible outer cover (3a) can be determined via the pressure of the pressure transmitter.

7. An apparatus in accordance with claim 6, **characterized in that** the flexible outer cover (3a) has a Shore hardness in the range between 20 and 90.

8. An apparatus in accordance with any one of the preceding claims, **characterized in that** the pressure or force measuring apparatus (7) includes a force transducer (2) and a flexible hollow body (7a) which extends in a straight line in the direction of extent (M) and which has an inner space (7b), **in that** the inner space (7b) of the hollow body of the pressure or force measuring apparatus is closed and includes a second liquid material (7h), and **in that** the force transducer (2) is coupled perpendicular to the direction of extent (M) toward the inner space (7b) of the hollow body of the pressure or force measuring apparatus, to measure the pressure of the second liquid material (7h).

9. An apparatus in accordance with any one of the claims 1 to 7, **characterized in that** the pressure or force measuring apparatus (7) includes a plurality of force transducers (2) which are arranged at the spacer element (3e) mutually spaced apart in the longitudinal direction (L).

10. An apparatus in accordance with any one of the preceding claims, **characterized in that** the lower fixed section (3d) forms part of a housing (6), **in that** the housing (6) has a standing surface (6b), and **in that** the lower fixed section (3d) is arranged such that the hollow body (3) extends substantially perpendicular to the standing surface (6b).

11. An apparatus in accordance with claim 1, **characterized in that** the pain actuator (10) is arranged at the housing (6).

12. Use of an apparatus in accordance with any one of the preceding claims for detecting perceptions of pain or the pain sensitivity of a person.

13. A method of detecting perceptions of pain or the pain intensity of a person, in that a cylindrical hollow body (3) is at least partly surrounded by a hand, in that a pressure is exerted onto a flexible outer cover (3a) of the hollow body (3) by pressing the hand closed, wherein the pressure is transmitted via a gel material, an elastic multicomponent material or a liquid material (4a, 4b) which acts as a pressure transmitter to a pressure or force measuring apparatus (7) arranged in the hollow body (3), wherein the elastic behavior of the flexible outer cover (3a) is set via the pressure of the pressure transmitter such that the diameter of the outer cover (3a) is maintained or is essentially maintained during the pressing, and wherein the pressure or the force measured by the pressure or force measuring apparatus (7) is used as a measure for the pain perceived by the person, and wherein a pain actuator (10) which heats up increasingly is touched, wherein the pain actuator includes a metallic cover at whose lower side a heating element and a temperature sensor are arranged, and wherein the measured pressure or the measured force is detected as a function of the temperature of the pain actuator (10).

## Revendications

1. Dispositif (1) de détection de la force manuelle ou de la pression manuelle et en particulier de sensations de douleur d'une personne, comprenant un dispositif de mesure de pression ou de force (7), et comprenant aussi un corps creux (3) s'étendant dans une direction longitudinale (L), dans lequel le corps creux (3) comprend une partie d'extrémité supérieure fixe (3c), une partie d'extrémité inférieure fixe (3d) ainsi qu'un élément de maintien de distance (3e), dans lequel la partie d'extrémité supérieure (3c) et la partie d'extrémité inférieure (3d) sont maintenues à distance l'une de l'autre par l'élément de maintien de distance (3e), dans lequel l'élément de maintien à distance (3e) s'étend en direction longitudinale (L), et dans lequel le corps creux (3) comprend une gaine extérieure flexible (3a), qui relie la partie d'extrémité supérieure (3c) à la partie d'extrémité inférieure (3d) de telle manière qu'il se forme un espace intérieur fermé (3b) à l'intérieur duquel l'élément de maintien de distance (3e) est également disposé, dans lequel la gaine extérieure (3a) est configurée de telle manière qu'elle puisse être au moins en partie enveloppée par une main, et dans lequel l'espace intérieur (3b) du corps creux (3) contient une matière de type gel, élastique à plusieurs composants ou liquide (4a), qui agit comme transmetteur de pression, et dans lequel le dispositif de mesure de pression ou de force (7) s'étend en direction longitudinale (L) au moins en partie dans l'espace intérieur (3b), afin de transmettre la pression de la gaine extérieure (3a) au dispositif de mesure de pression ou de force (7) par l'intermédiaire du transmetteur de pression, **caractérisé en ce que** le dispositif (1) comprend un déclencheur de douleur (10), qui est configuré comme un dispositif de production de chaleur, dans lequel le déclencheur de douleur (10) comprend un recouvrement métallique (10a), dont le côté inférieur est muni d'un élément chauffant (10c) ainsi que d'un détecteur de température (10b).

2. Dispositif (1) selon la revendication 1, **caractérisé en ce que** le dispositif de mesure de force (7) s'étend au moins le long de toute la longueur (L) de l'espace intérieur (3b).

3. Dispositif selon l'une quelconque des revendications précédentes, **caractérisé en ce que** le dispositif de mesure de pression ou de force (7) s'étend le long du centre du corps creux (3).

4. Dispositif selon l'une quelconque des revendications précédentes, **caractérisé en ce que** l'élément de maintien de distance (3e) est configuré sous forme de tube creux avec des ouvertures de paroi (3f), **en ce que** l'élément de maintien de distance (3e) s'étend le long de l'axe central du corps creux (3) et **en ce que** le dispositif de mesure de pression ou de force (7) est disposé en position étendue à l'intérieur de l'élément de maintien de distance (3e).

5. Dispositif selon l'une quelconque des revendications précédentes, **caractérisé en ce que** la gaine extérieure flexible (3a) est réalisée en forme cylindrique creuse.

6. Dispositif selon l'une quelconque des revendications précédentes, **caractérisé en ce que** la dureté ou la souplesse de la gaine extérieure flexible (3a) peut être déterminée par la pression du transmetteur de pression.

7. Dispositif selon la revendication 6, **caractérisé en ce que** la gaine extérieure flexible (3a) présente une dureté Shore située dans la plage de 20 à 90.

8. Dispositif selon l'une quelconque des revendications précédentes, **caractérisé en ce que** le dispositif de mesure de pression ou de force (7) comprend un enregistreur de force (2) ainsi qu'un corps creux flexible (7a) s'étendant en ligne droite dans la direction d'extension (M), avec un espace intérieur (7b), **en ce que** l'espace intérieur (7b) du corps creux du dispositif de mesure de pression ou de force est fermé et contient une deuxième matière liquide (7h), et **en ce que** l'enregistreur de force (2) est couplé perpendiculairement à la direction d'extension (M) en direction de l'espace intérieur (7b) du corps creux du dispositif de mesure de pression ou de force, afin de mesurer la pression de la deuxième matière liquide (7h).

9. Dispositif selon l'une quelconque des revendications 1 à 7, **caractérisé en ce que** le dispositif de mesure de pression ou de force (7) comprend une multiplicité d'enregistreurs de force (2), qui sont disposés à distance l'un de l'autre en direction longitudinale (L) sur l'élément de maintien de distance (3e).

10. Dispositif selon l'une quelconque des revendications précédentes, **caractérisé en ce que** la partie inférieure fixe (3d) fait partie d'un boîtier (6), **en ce que** le boîtier (6) présente une face de pose (6b), et **en ce que** la partie inférieure fixe (3d) est disposée de telle manière que le corps creux (3) soit essentiellement perpendiculaire à la face de pose (6b).

11. Dispositif selon la revendication 1, **caractérisé en ce que** le déclencheur de douleur (10) est disposé sur le boîtier (6).

12. Utilisation d'un dispositif selon l'une quelconque des revendications précédentes pour la détection de sensations de douleur ou de la sensibilité à la douleur d'une personne.

13. Procédé de détection de sensations de douleur ou de la sensibilité à la douleur d'une personne, dans lequel on enveloppe au moins en partie avec une main un corps creux cylindrique (3), dans lequel on exerce par serrage de la main une pression sur une gaine extérieure flexible (3a) du corps creux (3), dans lequel la pression est transmise par une matière de type gel, élastique à plusieurs composants ou liquide (4a, 4b), qui agit comme transmetteur de pression, à un dispositif de mesure de pression ou de force (7) disposé dans le corps creux (3), dans lequel on règle le comportement élastique de la gaine extérieure flexible (3a) par la pression du transmetteur de pression, de telle manière que le diamètre de la gaine extérieure (3a) pendant la compression soit conservé ou soit essentiellement conservé, et en ce que l'on utilise la pression mesurée par le dispositif de mesure de pression ou de force (7) ou la force mesurée comme mesure de la douleur ressentie par la personne, et en ce que l'on touche un déclencheur de douleur (10) à chauffage croissant, qui comprend un recouvrement métallique, dont le côté inférieur est muni d'un élément chauffant ainsi que d'un détecteur de température, et en ce que l'on détecte la pression mesurée ou la force mesurée en fonction de la température du déclencheur de douleur (10).
